# EUROPEAN PATENT SPECIFICATION

(11) **EP 3 474 754 B1**
(45) Date of publication and mention of the grant of the patent: **28.10.2020**
(21) Application number: 17748921.8
(22) Date of filing: 28.06.2017
(51) Int. Cl.: A61B 17/064, A61B 17/06

(54) **SUTURE DEVICE**
NAHTVORRICHTUNG
DISPOSITIF DE SUTURE

(30) Priority: 28.06.2016 IT UA20164730
(43) Date of publication of application: 01.05.2019
(73) Proprietor: Promev S.r.l., 23900 Lecco (LC) (IT)
(72) Inventor: DALLOLIO, Villiam, 23900 Lecco (LC) (IT)
(74) Representative: Brunacci, Marco
(86) International application number: PCT/IB2017/053877
(87) International publication number: WO 2018/002846

(56) References cited:
- WO-A1-2006/052687
- US-A- 3 353 531
- US-A- 5 002 563
- US-A1- 2005 043 757
- US-A1- 2011 071 548
- US-A1- 2014 046 366
- US-A1- 2014 058 411

## Description

### Technical Field

The present invention relates to a suture device.

### Background Art

In the surgical field, numerous types of suture devices are known which vary according to the different types of surgical, micro-surgical and endoscopic (e.g., laparoscopy, brain endoscopy, base of skull surgery, arthroscopy) procedures. With particular reference to endoscopy surgery, access to the operating field is normally carried out by means of the use of one or more access paths obtained directly in the patient's body or through one or more cannulas inserted through small-size cuts.

In the above type of surgical operations, the operating field is only accessible through one access path with very reduced dimensions or through a cannula.

It is easy to appreciate how in this type of operation, making sutures is particularly complex and laborious; for example, in a number of cases, the surgeon creates the passage of the suture device through the tissue to be sutured, by forming a surgical knot outside the surgical wound and then moving the latter, by means of a pusher element passing along the access path or the cannula, in a position adjacent to the tissue to be sutured.

The known devices used in such a procedure comprise a suture element with an extremity provided with a hollow needle element, and guide means of the type of a loop made of metal material and adapted to guide the suture device through the tissue to be sutured.

Such type of device is described in patent document US 5,755,728 wherein, in detail, the suture element comprises roller means adapted to allow it to pass through the tissue to be sutured.

Another type of known device is described in patent document US 6,514,265 wherein the suture element has an extremity associated with a needle element adapted to pass through the edges of the tissue to be sutured.

In this type of device, the suture is closed by radially pressing the suture element, thereby determining its transition from an initial configuration wherein the suture element has its extremities moved away from each other, and a final configuration wherein the suture element itself has its extremities closer to each other.

In the case in question, the suture element is provided with spring locking means which maintain it in initial configuration and cause it to pass to the final configuration.

A further type of known device is described in patent document US 5,002,563, wherein the suture element is made of thermally deformable material and has an extremity associated with a needle element adapted to pass through the flaps of the surgical wound.

The suture element, after having been positioned astride of the flaps of the surgical wound, is heated in such a way as to determine its transition from the initial configuration to the final configuration.

Nevertheless, the known devices have a number of drawbacks among which must be included the fact that the surface of the suture element is porous; this leads to a considerable increase in the risks of infection and inflammatory reactions.

Another drawback is tied to the presence of suture knots, the formation of which, being done manually, involves differences in terms of the strength of the suture stitches.

In the case in question, the presence of knots determines the presence of slacker and therefore less strong suture stitches, and tighter and more constraining suture stitches which often cause pain and ischemia.

To this must be added the fact that the presence of suture knots considerably increases the scar reaction phenomenon.

Another drawback is tied to the presence of the needle element, the latter involving an enlargement of the passage hole through which the suture element is made to pass; this considerably increases the risk of infections and reduces the strength of the suture stitches.

Furthermore, the presence of the needle element increases the risk of accidental pricking by the operators in the field, increasing the risk of infections and, furthermore, determining a huge waste of material tied to the need to use numerous auxiliary instruments during the suture.

Moreover, the presence of the needle elements makes it difficult and complicated to handle the suture device. In detail, the presence of the needle element prevents performing the suture and closing the latter in a continuous way. In fact, once the needle has been made to pass through the wound flaps, it must be removed and this produces yet another far from negligible waste of material.

To this must be added the fact that the need to remove the needle element causes an interruption of the suture procedure; the operator must in fact remove the needle element, taking his eyes away of the suture in order to grip a heating device adapted to determine the thermal deformation of the suture element and the closing on itself of the latter.

It is easy to appreciate that the interruption of the suture procedure together with the need to operate various instruments, besides determining a considerable increase in operating times, also increases the risk of error with direct consequences on the healing times of the suture.

### Description of the Invention

The main aim of the present invention is to provide a suture device that permits considerably reducing the risk of infections and of inflammatory reactions tied to the presence of the suture stitches.

Another object of the present invention is to provide a suture device which permits reducing the waste of material and the risk of contamination by the operators in the field.

A further object of the present invention is to provide a suture device which permits making suture stitches through an easy procedure, which is also simple to carry out, avoiding having to make any knots, and wherein the strength of the suture stitches is uniform.

Yet another object of the present invention is to provide a suture device which permits making sutures with a single instrument, eliminating the need to make use of different instruments such as, e.g. needles and heating devices.

Another object of the present invention is to provide a suture device which allows to overcome the aforementioned drawbacks of the prior art within the ambit of a simple, rational, easy, efficient to use and cost-effective solution.

The aforementioned objects are achieved by this suture device having the characteristics of claim 1.

### Brief Description of the Drawings

Other characteristics and advantages of the present invention will become more evident from the description of a preferred, but not exclusive, embodiment of a suture device, illustrated by way of an indicative, but non-limiting example, in the attached drawings in which:
Figure 1 is a front view of the device according to the invention in a first embodiment;
Figure 2 is a front view of the device according to the invention in a second embodiment;
Figure 3 is a front view of the device according to the invention in a third embodiment;
Figure 4 is a front view of the device according to the invention in a fourth embodiment;
Figure 5 is a front view of the device according to the invention in a fifth embodiment;
Figure 6 is a schematic representation of the device of Figure 5 in the operating mode;
Figures 7-8 are schematic representations of the device of Figure 3 in the operating mode;
Figure 11 is an axonometric view of the device of Figure 4;
Figure 12 is an axonometric view of the kit support device not forming part of the present invention;
Figure 13 is an axonometric view of the kit support device not forming part of the present invention.

### Embodiments of the Invention

With particular reference to these figures, reference numeral 1 globally indicates a suture device.

The device 1 comprises a suture element 2 of elongated shape and intended to be inserted through the flaps 3 of a surgical wound.

According to the invention, the suture element 2 is made of a shape memory material and has a first pointed extremity 4 adapted to pierce the flaps 3 of the surgical wound to allow the passage of the suture element 2 through the latter and their mutual approach to define a suture 5, and a second extremity 11 opposite to the first extremity itself.

Specifically, the shape memory material comprises Nitinol.

In this respect, the presence of titanium oxide represents a protective cover which prevents the release of nickel ions.

Again, according to the invention, the suture element 2, being made of a shape memory material, is movable, by heating, between:
- an initial configuration, wherein the suture element itself is fitted through the flaps 3 following the piercing of the latter with the first extremity 4, and wherein the extremities 4, 11 and the flaps 3 are moved away from each other; and
- a final configuration, wherein the suture element 2 is fitted astride of the flaps 3, and has the extremities 4, 11 and the flaps reciprocally moved closer to define a suture 5.

The first extremity 4 can be shaped differently, e.g., it may present a pyramid, spatula or duckbill shape.

Preferably, the suture element 2 has a length between 2 mm and 30 mm. Advantageously, the suture element 2 has a length between 4.5 mm and 17 mm. Preferably, the suture element 2 has a cross section greater than 100 µm. Conveniently, the suture element 2 has a cross section between 200 µm and 500 µm.

Advantageously, the suture element 2 has a substantially circular cross section. Furthermore, alternative embodiments cannot be ruled having the suture element 2 with cylindrical, triangular or cutting triangular cross section.

It is worth specifying that within the present treatise, the expression cross section is meant with reference to the diameter of the section of the suture element 2.

The suture element 2 comprises a curved portion 6 intended to be positioned astride of the flaps 3.

The curved portion 6 has a first end 7 associated with a first extension 14 and a second end 9 associated with a second extension 15 which, at the end of the suture, i.e., in the final configuration described in detail in the continuation of the present treatise, are adapted to overlap to each other to define a reinforcing surface 16 of the suture element 2.

More in detail, the first extremity 4 and the second extremity 11 are made on the first extension 14 and on the second extension 15 respectively, which in the final configuration define precisely the reinforcing surface 16 of the suture element 2.

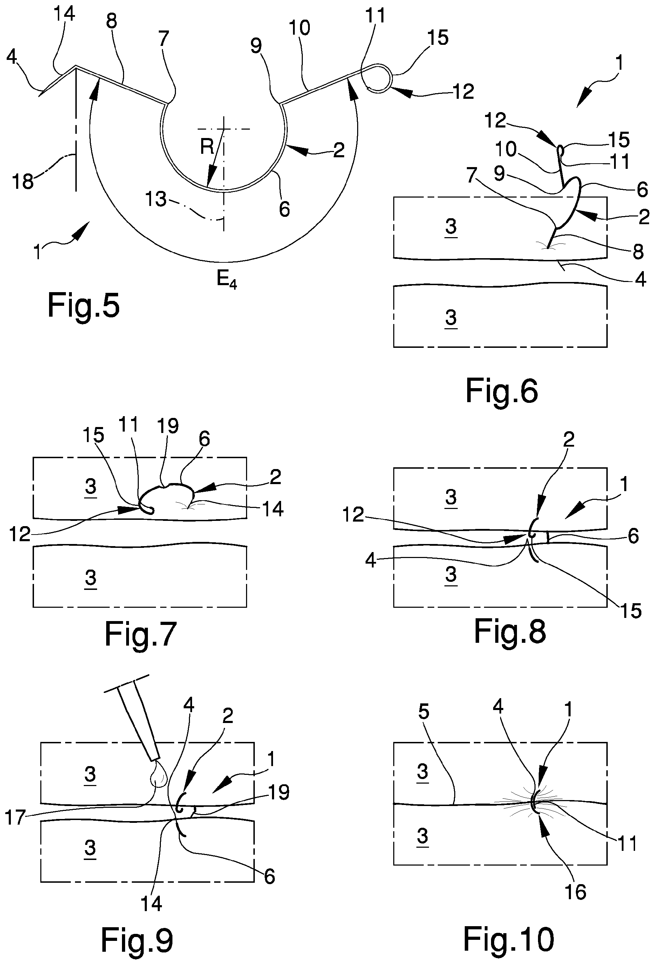

In the figures 5 and 6 an alternative embodiment is shown wherein the first end 7 and the second end 9 are associated with a first length 8 of the suture element 2 and with a second length 10 of the suture element respectively.

The first length 8 joins the first end 7 to the first extension 14 and, parallel, the second length 10 joins the second end 9 to the second extension 15.

In order to facilitate the penetration of the suture element 2, the first extension 14, in this case the first extremity 4, is inclined with respect to a vertical axis 18 passing through the first end 7 by an angle substantially equal to 30°. Preferably, the vertical axis 18 is the orthogonal projection of the first end 7.

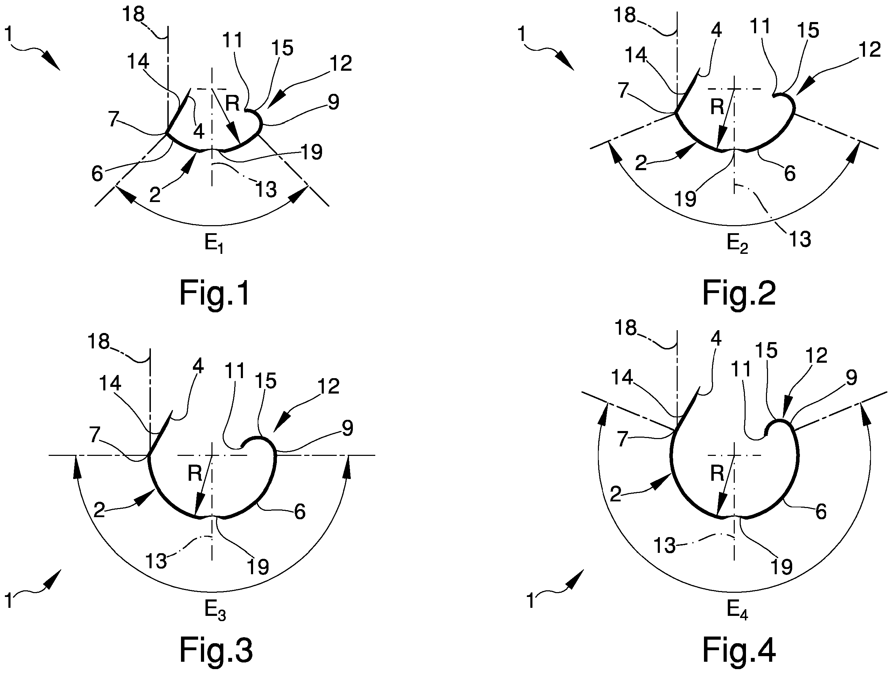

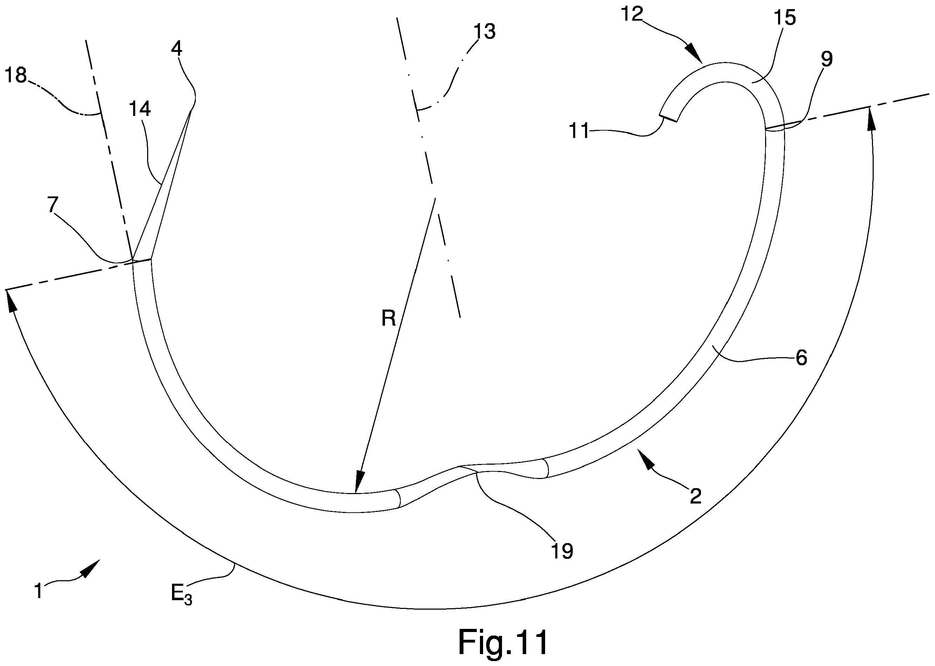

In detail, the first extremity 4 is oriented towards the inside of the curved portion 6 (figures 1-4 and 7-11).

Alternative embodiments cannot furthermore be ruled out having the first extremity 4 oriented towards the outside of the curved portion 6 (figures 5 and 6).

The second extremity 11 is provided with a locking portion 12 to lock the curved portion 6 astride of the flaps 3.

The locking portion 12 has a curved profile wound at least in part around itself and adapted to prevent the second length 10 from slipping out of the flaps 3.

As can be seen in the figures, the locking portion 12 is substantially hook shaped.

Moreover, the curved portion 6 comprises a flat-shaped section 19.

The flat-shaped section 19 is adapted to reduce the curvature in the external direction with respect to a central axis 13 passing through the curved portion 6. At the same time, the aforementioned flat-shaped section 19 is adapted to facilitate the gripping of the device 1 by means of surgical instruments, such as, e.g., a support device 20 of the suture device according to the present invention. With reference to the particular embodiment shown in the figures, the first extremity 4 and the second extremity 11 are formed on the first extension 14 and on the second extension 15, respectively.

Alternative embodiments cannot also be ruled out wherein the extensions 14, 15 are absent.

In this respect, it is pointed out that, with reference to the length of the suture element 2 is meant the extension presented by the suture element itself between the first end 7 and the second end 9, i.e., excluding the extensions 14, 15.

In fact, the first extension 14 and the second extension 15 have different extending measures according to the length of the suture element 2.

In the case in question, the extending measure of each extension 14, 15 is directly proportionate to the length of the suture element 2 by a factor substantially equal to 0.15.

To this must be added the fact that the first extension 14 and the second extension 15 have a module of different rigidity with respect to the curved portion 6.

More specifically, during manufacture, the extensions 14, 15 and the curved portion 6 are diversified from the viewpoint of thermal characterization.

In other words, the first extension 14 and the second extension 15 are manufactured in the austenitic phase and the curved portion 6 is manufactured in the martensitic phase.

Preferably, the curved portion 6 has a radius of curvature R greater than 0.5 mm.

It is specified that within this treatise, by the expression radius of curvature R is meant the radius of a circumference tangent to the curve defined by the curved portion 6, and which better approximates it.

Alternative embodiments cannot however be ruled out with the radius of curvature R greater than 2 mm.

Furthermore, alternative embodiments cannot be ruled out wherein the radius of curvature R has a gradual increase of 0.1 mm.

The curved portion 6 is adapted to contain the flaps 3 of the tissue to be sutured inside the relative radius of curvature R.

The aforementioned values are therefore variable according to the physiognomic characteristics of the tissue, such as, e.g., thickness, distance of introduction of the suture element 2 with respect to the flaps 3.

To this end, the curved portion 6 has a plurality of angular extensions E₁, E₂, E₃, E₄ different the one from the other according to the above-mentioned tissue characteristics.

In particular, within this treatise, by the expression "angular extension" E₁, E₂, E₃, E₄ is meant the length expressed in sexagesimal degrees around the central axis 13 of the curved portion 6 and defined by the angular distance between the first end 7 and the second end 9 respectively.

The curved portion 6 has an angular extension E₁, E₂, E₃, E₄ between 90° and 225°.

With reference to the figures 1-4 four embodiments are shown of the device 1 wherein the curved portion 6 has four different angular extensions E₁, E₂, E₃, E₄. In a first embodiment of the device 1, the curved portion 6 has an angular extension E₁ substantially equal to 90° (Figure 1).

In a second embodiment of the device 1, the curved portion 6 has an angular extension E₂ substantially equal to 135° (Figure 2).

In a third embodiment of the device 1, the curved portion 6 has an angular extension E₃ substantially equal to 180° (Figure 3).

In a fourth embodiment of the device 1, the curved portion 6 has an angular extension E₄ substantially equal to 225° (Figure 4).

As is known to the expert in the sector, shape memory material is super-elastic: this permits the transition of such material between two different crystalline shapes, the transition of which from one phase to another is due to various work temperature values.

As said above, the shape memory material is thermally deformable at a work temperature comprised between 10°C and 40°C.

In the case in question, the suture element 2 is movable by heating between:
- the initial configuration (figure 8) wherein the extremities 4, 11 are spaced apart from one another and the work temperature is below 20°C; and
- the final configuration (figure 10) wherein the extremities 4, 11 are sealed together to reclose the curved portion 6, the flaps 3 are approached to each other to define the suture 5 and the work temperature is higher than 30°C.

In particular, in the initial configuration the work temperature is comprised between 16°C and 20°C, and in the final configuration the work temperature is comprised between 36°C and 40°C.

Preferably, in the initial configuration, the work temperature is substantially equal to 18°C.

Advantageously, in the final configuration the work temperature is substantially equal to 38°C.

With reference to the particular embodiment shown in the figures, heating occurs by means of the irrigation of a fluid 17 on the flaps 3.

Preferably, such fluid 17 is water.

Alternative embodiments cannot however be ruled out having other types of heating, e.g. of the electric type.

It is worth specifying that the shape memory material in the initial configuration is in the martensitic phase, and in the final configuration is in the austenitic phase.

More in detail, the martensitic phase is stable at low temperatures and, on the contrary, the austenitic phase is stable at high temperatures.

The maintenance of the suture element 2 in the final configuration is ensured by the physiological temperature of the human body.

In fact, in case of the removal of the suture element 2, the latter is irrigated with a fluid 17 at a work temperature substantially equal to 15°C aimed at returning the suture element 2 to the initial configuration.

Before the detailed explanation of the operation of the present invention, it is useful to underline that the device 1 is compatible with Nuclear Magnetic Resonance (NMR) examinations.

Furthermore, the presence of a shape memory material permits graduating the transition phases from the initial configuration to the final configuration, by providing intermediate work temperatures.

In other words, it is possible to intervene on the closure of the suture element, graduating the work temperatures and obtaining a millimeter closure gradient. The operation of the present invention is the following.

In the face of a surgical wound, the flaps 3 are spaced apart from one another (figures 6 and 7) and the first extremity 4 of the suture element 2 is made to pass through the two flaps 3 so as to position the curved portion 6 astride of them (figure 8), thereby defining the initial configuration.

In the case in question, in the initial configuration the ends 7, 9 are spaced apart from one another and the curved portion 6 encircles the tissue to be sutured inside it.

The positioning in initial configuration is ensured by the presence of the locking portion 12 which prevents the second extension 15 from overrunning the tissue. The radius of curvature R and the angular extension E₁, E₂, E₃, E₄ are selected by the operator in the field according to the characteristics of the tissue to be sutured, e.g., according to its thickness or hardness.

After the curved portion 6 has been positioned, the suture element 2 is heated by means of the irrigation of water 17 at a work temperature substantially equal to 38°C; this determines the transition of the shape memory material from the martensitic phase to the austenitic phase.

In the transition from the initial configuration to the final configuration (figure 10), the extensions 14, 15 overlap to each other in an out of axis way to define the reinforcing surface 16.

The two extensions 14, 15, in exceeding the junction points of the ends 7, 9 considerably increase the strength of the suture element 2 in the final configuration.

Given the need to remove the suture element 2, the latter is irrigated by means of water at a work temperature substantially equal to 15°C; this causes the return of the suture element 2 to the initial configuration, i.e., in the martensitic phase.

The extremities 4, 11, by moving away from each other, interrupt the reinforcing surface 16, making it possible to remove the suture element 2 from the flaps 3.

Furthermore, the present disclosure also relates to a suture kit 1, 20.

The kit 1, 20 comprises the suture device 1 described previously and to the detailed description of which full reference is made.

Furthermore, the kit 1, 20 comprises a support device 20 of the device 1. Specifically, the support device 20 comprises a main body 22, 23 having a gripping portion 22 graspable by an operator and a fastening portion 23 of the suture device itself.

The gripping portion 22 comprises a first arm 22a and a second arm 22b associated with each other by interposition of connection means 22c of the type of a screw or a spring.

In other words, the support device 20 is based on a double lever of first class, like scissors in common use, wherein the connection means 22c act as a fulcrum.

In the same way, the fastening portion 23 comprises a beak element 24 movable between an opening configuration wherein the first arm 22a and the second arm 22b are spaced apart from each other, and a closing configuration wherein the first arm 22a and the second arm 22b are approached to one another.

In other words, the operator, by gripping the support device 20, grasps the suture device 1 determining the transition from the opening configuration, wherein the beak element 24 is also open, to the closing configuration, wherein the beak element 24 is closed and retains the suture device itself.

Furthermore, the support device 20 comprises heating means adapted to induce a change in temperature at the suture device 1.

More in detail, the heating means comprise a duct 25 passing through the main body 22, 23.

With reference to a particular embodiment, the heating means comprise a heating element passing through the duct 25 and associable with power supply means.

In this specific case, the heating means are of the electric type.

According to an alternative embodiment, the heating means comprise a heating element of the type of a fluid passing through the duct 25.

Preferably, the through duct is provided with an entry gap 27 made at the gripping portion 22, in this case either on the first arm 22a or on the second arm 22b, and associable with fluid feeding means, and with an exit gap 28 made at the fastening portion 23 and adapted to allow the dispensing of the fluid at the suture 5.

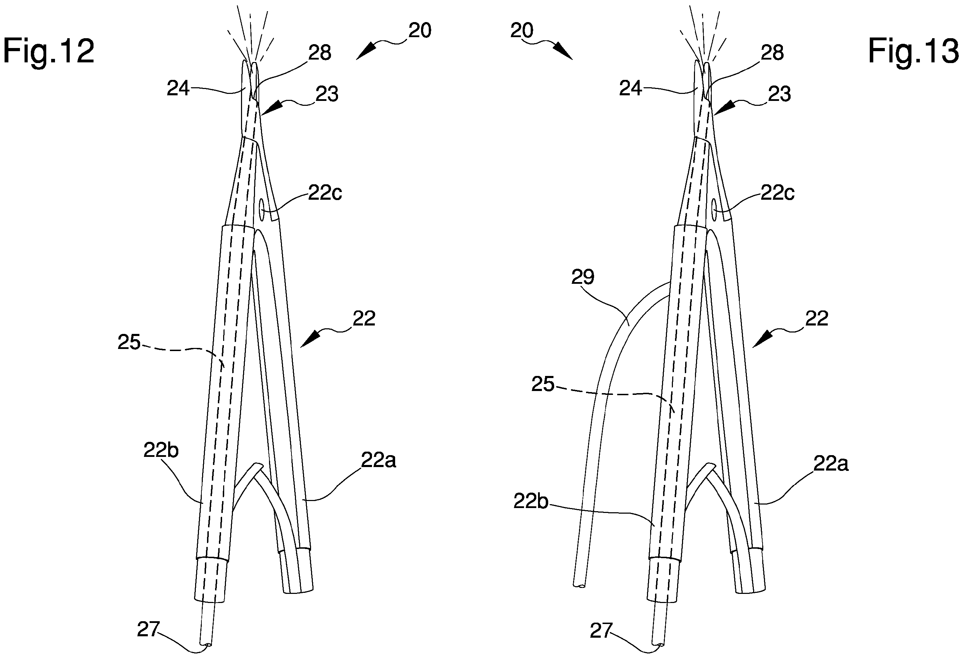

As shown in Figure 13, the presence cannot be ruled out of an auxiliary duct 29 associable with an entry hole and communicating with the duct 25.

The heating means are operatively connected to activation means of the type of a pedal which can be pressed as required by the operator.

The operation of the kit 1, 20 is as follows.

The operator grips the support device 20, grasping the suture device 1 with it.

In detail, the operator grips the support device 20 in the opening configuration, i.e., with the first arm 22a and the second arm 22b spaced apart from each other, and grasps the suture device 1 by means of the reciprocal approaching of the first and of the second arm 22a, 22b which causes the closure of the beak element 24.

At this point, the suture device 1 is made to pass through the flaps 3 and, after having been positioned, it is heated by means of the activation of the heating means.

In other words, the transition from the martensitic phase to the austenitic phase of the suture element 2 takes place by means of diffused heating at the suture 5. This means that after the suture element 2 has been positioned, the operator, by pressing on the activation means, diffuses heat, by means of the dispensing of the heating fluid or electricity.

It has in practice been ascertained that the described invention achieves the intended objects.

The fact is underlined that the particular solution of providing a suture element having a pointed extremity adapted to pierce the flaps of the surgical wound, and made of Nitinol, permits making a suture completely without knots, while at the same time eliminating the use of needles and considerably reducing the risk of infections and inflammations.

To this the fact must be added that the presence of a shape memory material permits making a completely operator-independent and needle-independent suture, wherein the closure of the suture element is determined only by the applied work temperature, ensuring the formation of a suture the strength of which is uniform over the entire extension surface of same.

## Claims

1. Suture device (1), comprising at least a suture element (2) of elongated shape and intended to be inserted through the flaps (3) of a surgical wound, wherein said suture element (2) is made of a shape memory material and has a first pointed extremity (4) adapted to pierce said flaps (3) to allow the passage of said suture element (2) through the latter , and a second extremity (11) opposite to said first extremity (4), and comprises at least a curved portion (6) intended to be positioned astride of said flaps (3), and wherein said suture element (2) comprises an initial configuration wherein said extremities (4, 11) and said flaps (3) are spaced apart from one another and a final configuration wherein said extremities (4, 11) and said flaps (3) are approached, wherein said first extremity (4) and said second extremity (11) are formed on a first extension (14) and on a second extension (15) respectively, which in said final configuration are adapted to overlap to each other to define a reinforcing surface (16) of said suture element (2), **characterized in that** said first extension (14) and said second extension (15) are manufactured in the austenitic phase and said curved portion (6) is manufactured in the martensitic phase.

2. Device (1) according to claim 1, **characterized by** the fact that said shape memory material comprises Nitinol.

3. Device (1) according to one or more of the preceding claims, **characterized by** the fact that said suture element (2) has a length between 2 mm and 30 mm.

4. Device (1) according to one or more of the preceding claims, **characterized by** the fact that said suture element (2) has a length between 4.5 mm and 17 mm.

5. Device (1) according to one or more of the preceding claims, **characterized by** the fact that said suture element (2) has a cross section greater than 100 µm.

6. Device (1) according to one or more of the preceding claims, **characterized by** the fact that said curved portion (6) has a radius of curvature (R) greater than 0.5 mm.

7. Device (1) according to one or more of the preceding claims, **characterized by** the fact that said curved portion (6) has an angular extension (E₁, E₂, E₃, E₄) between 90° and 225°.

8. Device (1) according to one or more of the preceding claims, **characterized by** the fact that said second extremity (11) is provided with a locking portion (12) to lock said curved portion (6) astride of said flaps (3).

9. Device (1) according to one or more of the preceding claims, **characterized by** the fact that said shape memory material is thermally deformable at a work temperature comprised between 10°C and 40°C.

10. Device (1) according to one or more of the preceding claims, **characterized by** the fact that:
- in said initial configuration, wherein said extremities (4, 11) are spaced apart from one another, said work temperature is below 20°C; and
- in said final configuration, wherein said extremities (4, 11) are moved close together to reclose said curved portion (6), said flaps (3) are approached to each other to define said suture (5), said work temperature is higher than 30°C.

11. Device (1) according to one or more of the preceding claims, **characterized by** the fact that in said initial configuration said work temperature is comprised between 16°C and 20°C, and in said final configuration said work temperature is comprised between 36°C and 40°C.

12. Device (1) according to one or more of the preceding claims, **characterized by** the fact that said shape memory material of the curved portion (6) in said initial configuration is in the martensitic phase, and in said final configuration is in the austenitic phase.

13. Device (1) according to one or more of the preceding claims, **characterized by** the fact that said curved portion (6) comprises at least a flat-shaped section (19).

14. Device (1) according to one or more of the preceding claims, **characterized by** the fact that said curved portion (6) comprises a first end (7) associated with said first extension (14) and a second end (9) associated with said second extension (15), said first extension (14) being inclined with respect to a vertical axis (18) passing through said first end (7) by an angle substantially equal to 30°.

15. Device (1) according to one or more of the preceding claims, **characterized by** the fact that said first extension (14) and said second extension (15) have a module of different rigidity with respect to said curved portion (6).

## Patentansprüche

1. Nahtvorrichtung (1), umfassend mindestens ein Nahtelement (2) von länglicher Form, das dazu bestimmt ist, durch die Klappen (3) einer chirurgischen Wunde eingeführt zu werden, wobei das Nahtelement (2) aus einem Formgedächtnismaterial hergestellt ist und eine erste spitze Extremität (4) aufweist, das geeignet ist, die Klappen (3) zu durchstechen, um den Durchgang des Nahtelements (2) durch letztere zu ermöglichen, und eine zweite Extremität (11), das der ersten Extremität (4) gegenüberliegt, und mindestens einen gekrümmten Abschnitt (6) aufweist, der rittlings auf den Klappen (3) positioniert werden soll, und wobei das Nahtelement (2) eine Anfangskonfiguration aufweist, bei der die Extremitäten (4, 11) und die Klappen (3) voneinander beabstandet sind und eine Endkonfiguration, bei der die Extremitäten (4, 11) und die Klappen (3) einander angenähert sind, wobei die erste Extremität (4) und die zweite Extremität (11) auf einer ersten Verlängerung (14) beziehungsweise auf einer zweiten Verlängerung (15) ausgebildet sind, die in der Endkonfiguration so angepasst sind, dass sie einander überlappen, um eine Verstärkungsfläche (16) des Nahtelements (2) zu definieren, **dadurch gekennzeichnet, dass** die erste Verlängerung (14) und die zweite Verlängerung (15) in der austenitischen Phase hergestellt sind und der gekrümmte Abschnitt (6) in der martensitischen Phase hergestellt ist.

2. Vorrichtung (1) nach Anspruch 1, **gekennzeichnet durch** die Tatsache, dass das Formgedächtnismaterial Nitinol enthält.

3. Vorrichtung (1) nach einem oder mehreren der vorstehenden Ansprüche, **gekennzeichnet durch** die Tatsache, dass das Nahtelement (2) eine Länge zwischen 2 mm und 30 mm aufweist.

4. Vorrichtung (1) nach einem oder mehreren der vorstehenden Ansprüche, **gekennzeichnet durch** die Tatsache, dass das Nahtelement (2) eine Länge zwischen 4,5 mm und 17 mm aufweist.

5. Vorrichtung (1) nach einem oder mehreren der vorstehenden Ansprüche, **gekennzeichnet durch** die Tatsache, dass das Nahtelement (2) einen Querschnitt von mehr als 100 µm aufweist.

6. Vorrichtung (1) nach einem oder mehreren der vorstehenden Ansprüche, **gekennzeichnet durch** die Tatsache, dass der gekrümmte Abschnitt (6) einen Krümmungsradius (R) von mehr als 0,5 mm aufweist.

7. Vorrichtung (1) nach einem oder mehreren der vorstehenden Ansprüche, **gekennzeichnet durch** die Tatsache, dass der gekrümmte Abschnitt (6) eine Winkelausdehnung (E₁, E₂, E₃, E₄) zwischen 90° und 225° aufweist.

8. Vorrichtung (1) nach einem oder mehreren der vorhergehenden Ansprüche, **gekennzeichnet durch** die Tatsache, dass die zweite Extremität (11) mit einem Verriegelungsabschnitt (12) versehen ist, um den gekrümmten Abschnitt (6) rittlings auf den Klappen (3) zu verriegeln.

9. Vorrichtung (1) nach einem oder mehreren der vorstehenden Ansprüche, **gekennzeichnet durch** die Tatsache, dass das Formgedächtnismaterial bei einer Arbeitstemperatur zwischen 10°C und 40°C thermisch verformbar ist.

10. Vorrichtung (1) nach einem oder mehreren der vorstehenden Ansprüche, **gekennzeichnet durch** die Tatsache, dass
- in der Anfangskonfiguration, in der die Extremitäten (4, 11) voneinander beabstandet sind, die Arbeitstemperatur unter 20°C liegt; und
- in der Endkonfiguration, wobei die Extremitäten (4, 11) nahe beieinander bewegt werden, um den gekrümmten Abschnitt (6) wieder zu schließen, die Klappen (3) einander angenähert werden, um die Naht (5) zu definieren, die Arbeitstemperatur höher als 30°C ist.

11. Vorrichtung (1) nach einem oder mehreren der vorhergehenden Ansprüche, **gekennzeichnet durch** die Tatsache, dass in der Anfangskonfiguration die Arbeitstemperatur zwischen 16°C und 20°C liegt und in der Endkonfiguration die Arbeitstemperatur zwischen 36°C und 40°C liegt.

12. Vorrichtung (1) nach einem oder mehreren der vorhergehenden Ansprüche, **gekennzeichnet durch** die Tatsache, dass das Formgedächtnismaterial des gekrümmten Abschnitts (6) sich in der Anfangskonfiguration in der martensitischen Phase und in der Endkonfiguration in der austenitischen Phase befindet.

13. Vorrichtung (1) nach einem oder mehreren der vorstehenden Ansprüche, **gekennzeichnet durch** die Tatsache, dass der gekrümmte Abschnitt (6) mindestens einen flach geformten Bereich (19) aufweist.

14. Vorrichtung (1) nach einem oder mehreren der vorstehenden Ansprüche, **gekennzeichnet durch** die Tatsache, dass der gekrümmte Abschnitt (6) ein erstes Ende (7), das mit einer ersten Verlängerung (14) verbunden ist, und ein zweites Ende (9), das mit der zweiten Verlängerung (15) verbunden ist, aufweist, wobei die erste Verlängerung (14) in Bezug auf eine vertikale Achse (18), die durch das erste Ende (7) verläuft, um einen Winkel von im Wesentlichen gleich 30° geneigt ist.

15. Vorrichtung (1) nach einem oder mehreren der vorhergehenden Ansprüche, **gekennzeichnet durch** die Tatsache, dass die erste Verlängerung (14) und die zweite Verlängerung (15) ein Modul unterschiedlicher Steifigkeit in Bezug auf den gekrümmten Abschnitt (6) aufweisen.

## Revendications

1. Dispositif de suture (1), comprenant au moins un élément de suture (2) de forme allongée et destiné à être inséré à travers les lambeaux (3) d'une plaie chirurgicale, dans lequel ledit élément de suture (2) est réalisé en matériau à mémoire de forme et a une extrémité pointue (4) adaptée pour percer lesdits lambeaux (3) pour permettre le passage dudit élément de suture (2) à travers ceux-ci, et une seconde extrémité (11) opposée à ladite première extrémité (4), et comprend au moins une partie incurvée (6) destinée à être positionnée à cheval sur lesdits lambeaux (3), et dans lequel ledit élément de suture (2) comprend une configuration initiale dans laquelle lesdites extrémités (4, 11) et lesdits lambeaux (3) sont espacés l'un de l'autre et une configuration finale dans laquelle lesdites extrémités (4, 11) et lesdits lambeaux (3) sont approchés, dans lequel ladite première extrémité (4) et ladite seconde extrémité (11) sont formées sur une première extension (14) et sur une seconde extension (15) respectivement, qui dans ladite configuration finale sont adaptées pour se chevaucher l'une l'autre pour définir une surface de renforcement (16) dudit élément de suture (2), **caractérisé en ce que** ladite première extension (14) et ladite seconde extension (15) sont produites dans la phase austénitique et ladite partie incurvée (6) est produite dans la phase martensitique.

2. Dispositif (1) selon la revendication 1, **caractérisé par le fait que** ledit matériau à mémoire de forme comprend du nitinol.

3. Dispositif (1) selon l'une ou plusieurs des revendications précédentes, **caractérisé par le fait que** ledit élément de suture (2) a une longueur entre 2 mm et 30 mm.

4. Dispositif (1) selon l'une ou plusieurs des revendications précédentes, **caractérisé par le fait que** ledit élément de suture (2) a une longueur entre 4,5 mm et 17 mm.

5. Dispositif (1) selon l'une ou plusieurs des revendications précédentes, **caractérisé par le fait que** ledit élément de suture (2) a une section transversale supérieure à 100 µm.

6. Dispositif (1) selon l'une ou plusieurs des revendications précédentes, **caractérisé par le fait que** ladite partie incurvée (6) a un rayon de courbure (R) supérieur à 0,5 mm.

7. Dispositif (1) selon l'une ou plusieurs des revendications précédentes, **caractérisé par le fait que** ladite partie incurvée (6) a une extension angulaire (E₁, E₂, E₃, E₄) entre 90° et 225°.

8. Dispositif (1) selon l'une ou plusieurs des revendications précédentes, **caractérisé par le fait que** ladite seconde extrémité (11) est dotée d'une partie de verrouillage (12) pour verrouiller ladite partie incurvée (6) à cheval sur lesdits lambeaux (3).

9. Dispositif (1) selon l'une ou plusieurs des revendications précédentes, **caractérisé par le fait que** ledit matériau à mémoire de forme est déformable thermiquement à une température de travail comprise entre 10 °C et 40 °C.

10. Dispositif (1) selon l'une ou plusieurs des revendications précédentes, **caractérisé par le fait que** :
- dans ladite configuration initiale, dans laquelle lesdites extrémités (4, 11) sont espacées l'une de l'autre, ladite température de travail est inférieure à 20 °C ; et
- dans ladite configuration finale, dans laquelle lesdites extrémités (4, 11) sont rapprochées l'une de l'autre pour refermer ladite partie incurvée (6), lesdits lambeaux (3) sont approchés l'un de l'autre pour définir ladite suture (5), ladite température de travail est supérieure à 30 °C.

11. Dispositif (1) selon l'une ou plusieurs des revendications précédentes, **caractérisé par le fait que** dans ladite configuration initiale ladite température de travail est comprise ente 16 °C et 20 °C, et dans ladite configuration finale ladite température de travail est comprise entre 36 °C et 40 °C.

12. Dispositif (1) selon l'une ou plusieurs des revendications précédentes, **caractérisé par le fait que** ledit matériau à mémoire de forme de la partie incurvée (6) dans ladite configuration initiale est dans la phase martensitique, et dans ladite configuration finale est dans la phase austénitique.

13. Dispositif (1) selon l'une ou plusieurs des revendications précédentes, **caractérisé par le fait que** ladite partie incurvée (6) comprend au moins une section de forme plate (19).

14. Dispositif (1) selon l'une ou plusieurs des revendications précédentes, **caractérisé par le fait que** ladite partie incurvée (6) comprend une première extrémité (7) associée à ladite première extension (14) et une seconde extrémité (9) associée à ladite seconde extension (15), ladite première extension (14) étant inclinée par rapport à un axe vertical (18) passant à travers ladite première extrémité (7) selon un angle sensiblement égal à 30°.

15. Dispositif (1) selon l'une ou plusieurs des revendications précédentes, **caractérisé par le fait que** ladite première extension (14) et ladite seconde extension (15) ont un module de rigidité différente par rapport à ladite partie incurvée (6).
